(19) Europäisches Patentamt
European Patent Office
Office européen des brevets

(11) Numéro de publication : **0 564 333 A1**

(12) # DEMANDE DE BREVET EUROPEEN

(21) Numéro de dépôt : **93400784.0**

(51) Int. Cl.$^5$ : **C07C 37/60,** C07C 39/08

(22) Date de dépôt : **26.03.93**

(30) Priorité : **03.04.92 FR 9204061**

(43) Date de publication de la demande : **06.10.93 Bulletin 93/40**

(84) Etats contractants désignés : **DE FR GB IT NL SE**

(71) Demandeur : **RHONE-POULENC CHIMIE
25, quai Paul Doumer
F-92408 Courbevoie Cédex (FR)**

(72) Inventeur : **Costantini, Michel
10, rue du Dr Bonhomme
F-69003 Lyon (FR)**
Inventeur : **Laucher, Dominique
205, avenue Jean-Jaurès
F-69007 Lyon (FR)**

(74) Mandataire : **Dutruc-Rosset, Marie-Claude et al
RHONE-POULENC CHIMIE, Direction de la Propriété Industrielle, 25, quai Paul Doumer
F-92408 Courbevoie Cédex (FR)**

(54) **Procédé d'hydroxylation de composés phénoliques.**

(57) La présente invention a pour objet un procédé d'hydroxylation de composés phénoliques permettant d'accroître la quantité d'isomère para formée.

L'invention concerne un procédé d'hydroxylation de composés phénoliques au moyen de peroxyde d'hydrogène, en présence d'un catalyseur acide, ledit procédé étant caractérisé par le fait que la réaction est conduite en présence d'une quantité efficace d'un polymère porteur de groupes sulfoniques et d'un composé cétonique répondant à la formule générale (II) :

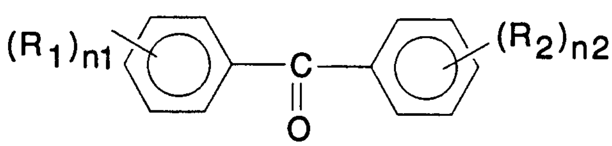

(II)

dans ladite formule (II) :

— $R_1$ et $R_2$ identiques ou différents représentent un atome d'hydrogène ou un groupe électro-donneur,

— $n_1$, $n_2$ identiques ou différents est un nombre égal à 0, 1, 2 ou 3,

EP 0 564 333 A1

La présente invention a pour objet un procédé d'hydroxylation de composés phénoliques et plus particulièrement un procédé d'hydroxylation de phénols et d'éthers de phénol, par le peroxyde d'hydrogène.

A l'heure actuelle, on est à la recherche d'un procédé d'hydroxylation du phénol en hydroquinone et pyrocatéchine qui conduise de manière prépondérante à l'hydroquinone.

Actuellement les procédés décrits dans l'état de la technique permettent d'obtenir principalement la pyrocatéchine.

Il s'avère que pour répondre à la demande du marché qui est fluctuante, il est important, de disposer d'un procédé industriel permettant d'augmenter la production d'hydroquinone formée par rapport à la quantité de pyrocatéchine.

Un deuxième problème qui se pose également est que les procédés qui sont couramment utilisés, font appel à une catalyse homogène.

Citons, entre autres, le brevet FR-A 2 071 464 qui concerne un procédé industriel très important d'hydroxylation de phénols et d'éthers de phénols.

Ledit procédé consiste à réaliser l'hydroxylation, par l'eau oxygénée, en présence d'un acide fort. Parmi ces acides forts, l'acide sulfurique, l'acide paratoluène-sulfonique, l'acide perchlorique sont les plus utilisés.

L'hydroxylation du phénol effectuée dans les conditions décrites conduit à un mélange d'hydroquinone et de pyrocatéchine, avec prédominance de celle-ci puisque le rapport hydroquinone/pyrocatéchine varie le plus souvent entre 0,3 et 0,7.

On obtient lors de la mise en oeuvre de ce procédé de la pyrocatéchine, en quantité majoritaire.

Bien que ce procédé soit très intéressant, il présente l'inconvénient de faire appel à une catalyse homogène. Il s'en suit un problème pour se débarrasser du catalyseur acide en fin de réaction. Pour éliminer l'acide, on peut [Ind. Eng. Chem. Prod. Res. Dev. 15, n°3 (1976)] séparer l'acide par un lavage aqueux puis traiter le milieu réactionnel par un mélange d'eau et d'éther isopropylique. L'acide reste en phase aqueuse et le phénol et les diphénols formés sont extraits dans l'éther isopropylique. Il est ensuite nécessaire de séparer par distillation l'éther isopropylique puis d'effectuer ensuite la distillation du phénol et des diphénols.

Par ailleurs, on connaît selon FR-A 2 266 683, un procédé qui consiste à effectuer l'hydroxylation du phénol, en présence d'une cétone. Il en résulte une amélioration du rendement de la réaction en hydroquinone et pyrocatéchine. Toutefois, tous les exemples décrits conduisent à une quantité plus importante de pyrocatéchine par rapport à celle d'hydroquinone.

Dans la demande de brevet FR-A 2 667 598, la demanderesse a proposé un procédé permettant d'accroître la quantité d'hydroquinone formée par rapport à la quantité de pyrocatéchine et d'obtenir dans sa variante préférentielle, plus d'hydroquinone que de pyrocatéchine.

Ledit procédé consiste à effectuer l'hydroxylation du phénol en présence d'une quantité efficace d'un acide fort, ledit procédé étant caractérisé par le fait que la réaction est conduite en présence d'un composé cétonique choisi parmi la benzophénone et les benzophénones dont les atomes d'hydrogène du noyau aromatique peuvent être substitués par un groupe électro-donneur.

Conformément au procédé décrit dans FR-A 2 667 598, la présence du composé cétonique tel que défini, lors de l'hydroxylation du phénol joue sur la régiosélectivité de la réaction et des rapports hydroquinone/pyrocatéchine variant entre 1,0 et 1,13 sont avantageusement obtenus.

Cependant, un tel procédé pose également le problème de l'élimination du catalyseur acide puisqu'il s'agit d'une catalyse homogène effectuée à l'aide d'un acide fort.

L'objectif de la présente invention est de résoudre le double problème qui consiste à fournir un procédé permettant d'accroître la quantité d'hydroquinone formée et de séparer facilement le catalyseur acide du milieu réactionnel à la fin de la réaction.

Un autre objectif de l'invention est de disposer d'un procédé d'hydroxylation du phénol faisant appel à une catalyse hétérogène et permettant d'obtenir plus d'hydroquinone que de pyrocatéchine.

Plus précisément, la présente invention a pour objet un procédé d'hydroxylation d'un composé phénolique présentant au moins un atome d'hydrogène en position para du groupe hydroxyle, par réaction dudit composé avec le peroxyde d'hydrogène, en présence d'un catalyseur acide, ledit procédé étant caractérisé par le fait que la réaction est conduite en présence d'une quantité efficace d'un polymère porteur de groupes sulfoniques et d'un composé cétonique répondant à la formule générale (II):

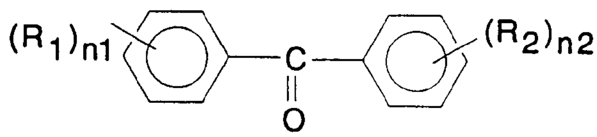

(II)

dans ladite formule (II) :

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position a par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

Une première caractéristique du procédé de l'invention est de faire appel à un système catalytique hétérogène qui est un polymère porteur de groupes sulfoniques.

Il existe sur le marché de nombreux polymères sulfoniques qui sont des résines commercialisées sous différentes dénominations commerciales. On peut citer, entre autres, les résines suivantes : TEMEX 50, AMBERLYST 15, AMBERLYST 35, AMBERLYST 36, DOWEX 50W.

Les résines précitées qui conviennent bien à la présente invention sont constituées d'un squelette polystyrénique qui porte des groupes fonctionnels qui sont des groupes sulfoniques.

Le squelette polystyrénique est obtenu par polymérisation du styrène et du divinylbenzène, sous l'influence d'un catalyseur d'activation, le plus souvent un peroxyde organique, ce qui conduit à un polystyrène réticulé. La polymérisation se fait le plus souvent en suspension et l'on obtient des billes ou des granules de polymère. Ceux-ci sont traités par de l'acide sulfurique ou sulfochlorique concentré. On obtient un copolymère styrène-divinylbenzène sulfoné.

Il est également possible de faire appel à des résines sulfoniques qui sont des copolymères phénol-formol et qui portent sur le noyau aromatique un groupe méthylène sulfonique. Comme exemples desdites résines, on peut mentionner entre autres, la résine commercialisée sous la dénomination DUOLITE ARC 9359.

D'autres résines sont également disponibles sur le marché et l'on peut citer les résines perfluorées porteuses de groupes sulfoniques et, plus particulièrement le NAFION qui présente la structure générale donnée ci-après:

$$[(CF_2 - CF_2)_n - CF - CF_2]_x$$
$$|$$
$$[OCF_2 - CF]_m\ OCF_2 - CF_2SO_3H$$
$$|$$
$$CF_3$$

dans ladite formule, m est un nombre entier égal à 1,2,3,---, n est compris entre 5 et 13,5 et x est égal à environ 1000.

Le NAFION est préparé à partir d'un copolymère de tétrafluoroéthylène et de perfluoro[2-(fluorosulfonyléthoxy)-propyl] vinyl éther.

Les résines en cause peuvent être du type gel ou du type macroréticulé. Elles sont mises en oeuvre sous la forme acide.

De nombreuses résines sont des produits disponibles sur le marché sous forme sèche ou humide. L'une ou l'autre des formes peuvent être utilisées dans le procédé de l'invention.

Elles se présentent habituellement sous forme de particules sensiblement sphériques ayant un diamètre variant de 0,3 à 1,5 mm, de préférence entre 0,5 et 1,2 mm.

Les résines précitées sont mises en oeuvre préférentiellement dans le procédé de l'invention et plus préférentiellement les résines ayant un squelette polystyrénique. Toutefois, l'invention n'exclut pas la mise en oeuvre de résines ayant un squelette d'une autre nature dans la mesure où elle porte les groupes sulfoniques adéquates.

La proportion de groupes sulfoniques par rapport à la masse polymérique peut être variable et l'on en tiendra compte lors de la détermination de la quantité de polymère à mettre en oeuvre.

La concentration en sites acides du polymère sulfonique varie avantageusement entre 1 et 10 milliéquivalents d'ions $H^+$ par gramme de polymère sec, et, de préférence, entre 2 et 7 milliéquivalents d'ions $H^+$ par gramme de polymère sec.

Une autre caractéristique du procédé de l'invention est de mettre en oeuvre un composé cétonique répondant à la formule précitée (II) qui est de préférence la benzophénone ou une benzophénone substituée par un groupe électro-donneur.

Dans l'exposé qui suit de la présente invention, on entend par 'groupe électro-donneur", un groupe tel que défini par H.C. BROWN dans l'ouvrage de Jerry MARCH - Advanced Organic Chemistry, chapitre 9, pages 243 et 244 (1985).

Conformément au procédé de l'invention, on choisit un groupe électro-donneur ne réagissant pas dans les conditions d'acidité de l'invention.

A titre d'exemples de groupes électro-donneurs convenant à la présente invention, on peut citer :
- les radicaux alkyle linéaires ou ramifiés ayant de 1 à 4 atomes de carbone,
- le radical phényle,
- les radicaux alkoxy $R_3$-O- dans lesquels $R_3$ représente un radical alkyle linéaire ou ramifié ayant de 1 à 4 atomes de carbone ou le radical phényle,
- le groupe hydroxyle,
- l'atome de fluor.

Parmi les composés cétoniques répondant à la formule générale (II), on fait appel tout particulièrement à ceux répondant à la formule générale (II) dans laquelle $R_1$ et $R_2$ identiques ou différents, représentent un atome d'hydrogène ou un groupe électro-donneur, de préférence en position 4,4' et $n_1$, $n_2$ identiques ou différents sont égaux à 0 ou 1.

On choisit préférentiellement les composés cétoniques répondant à la formule (II) dans laquelle $R_1$ et $R_2$, identiques ou différents, représentent un atome d'hydrogène ; un radical méthyle, éthyle, tertiobutyle, phényle ; un radical méthoxy ou éthoxy; un groupe hydroxyle, de préférence en position 3,3' ou 4,4'.

Comme exemples spécifiques de cétones qui peuvent être utilisées dans le procédé de l'invention, on peut citer plus particulièrement :
- la benzophénone
- la méthyl-2 benzophénone
- la diméthyl-2,4 benzophénone
- la diméthyl-4,4' benzophénone
- la diméthyl-2,2' benzophénone
- la diméthoxy-4,4' benzophénone
- la fluorénone
- l'hydroxy-4 benzophénone
- la dihydroxy-4-4' benzophénone
- la benzoyl-4 biphényle

Conformément au procédé de l'invention, on effectue l'hydroxylation d'un composé phénolique à l'aide de peroxyde d'hydrogène.

Dans l'exposé qui suit de la présente invention, on entend "par composé phénoliques", un composé aromatique dont un atome d'hydrogène directement lié au noyau aromatique est remplacé par un groupe hydroxyle et par "composé aromatique", la notion classique d'aromaticité telle que définie dans la littérature, notamment par Jerry MARCH, Advanced Organic Chemistry, 3ème édition, John Wiley and Sons, 1985, p.37 et suivantes.

La présente invention s'applique tout particulièrement au composé phénolique de formule générale (I):

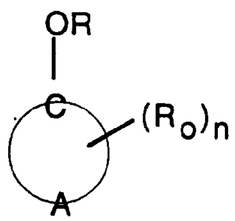

(I)

dans laquelle
- A symbolise un reste d'un radical carbocyclique aromatique, monocyclique ou polycyclique ou un radical divalent constitué par un enchaînement de deux ou plusieurs radicaux carbocycliques aromatiques monocycliques,

- R représente un atome d'hydrogène ou un radical hydrocarboné ayant de 1 à 24 atomes de carbone qui peut être un radical aliphatique saturé ou insaturé, linéaire ou ramifié ou un radical cycloaliphatique saturé ou insaturé ou aromatique, monocyclique ou polycyclique,
- $R_o$ représente un ou plusieurs substituants, identiques ou différents,
- n est un nombre inférieur ou égal à 4.

Le procédé de l'invention s'applique à tout composé phénolique répondant à la formule générale (I) et, plus particulièrement, aux composés phénoliques de formule (I) dans laquelle:

- le radical R représente l'un des groupes suivants :
  . un atome d'hydrogène
  . un radical alkyle linéaire ou ramifié ayant de 1 à 6 atomes de carbone,
  . un radical cyclohexyle,
  . un radical phényle,
  . un radical benzyle,
- le ou les radicaux $R_o$ représentent l'un des groupes suivants:
  . un atome d'hydrogène
  . un radical alkyle linéaire ou ramifié ayant de 1 à 6 atomes de carbone,
  . un radical alcényle linéaire ou ramifié ayant de 2 à 6 atomes de carbone,
  . un radical alkoxy du type $R_4$-O-, où $R_4$ représente un radical alkyle linéaire ou ramifié ayant de 1 à 6 atomes de carbone,
  . un groupe acyle ayant de 2 à 6 atomes de carbone,
  . un groupe hydroxyle,
  . un groupe $-COOR_5$, où $R_5$ représente un radical alkyle linéaire ou ramifié ayant de 1 à 4 atomes de carbone,
  . un atome d'halogène, de préférence, de fluor, de chlore, de brome,
  . un groupe $-CF_3$.
- n est un nombre égal à 0, 1, 2 ou 3.

Le composé phénolique de formule (I) peut être porteur d'un ou plusieurs substituants. Des exemples de substituants sont donnés ci-dessus mais cette liste ne présente aucun caractère limitatif. N'importe quel substituant peut être présent sur le cycle dans la mesure où il n'interfère pas au niveau du produit désiré.

Parmi les composés de formule (I), on met en oeuvre plus particulièrement ceux dont le reste (A) représente :

- un radical carbocyclique aromatique monocyclique ou polycyclique avec des cycles pouvant former entre eux un système orthocondensé répondant à la formule (Ia) :

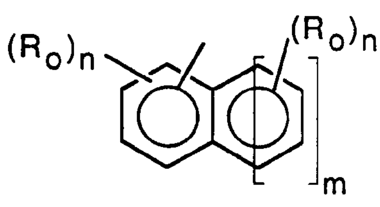

(Ia)

dans ladite formule (Ia), m représente un nombre égal à 0, 1 ou 2 et les symboles $R_o$ et n identiques ou différents ayant la signification donnée précédemment,

- un radical constitué par un enchaînement de deux ou plusieurs radicaux carbocycliques aromatiques monocycliques répondant à la formule (Ib) :

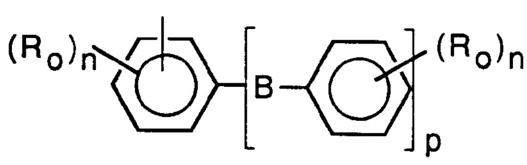

(Ib)

dans ladite formule (Ib), les symboles $R_o$ et n identiques ou différents ont la signification donnée précédemment, p est un nombre égal à 0, 1, 2 ou 3 et B représente :

- un lien valentiel
- un radical alkylène ou alkylidène ayant de 1 à 4 atomes de carbone, de préférence, un radical méthylène ou isopropylidène,
- un des groupes suivants:
  -O-, -COO-, -OCOO-,
  $-SO_2$,

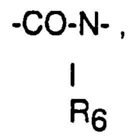

dans ces formules, $R_6$ représente un atome d'hydrogène ou un radical alkyle ayant de 1 à 4 atomes de carbone, un radical cyclohexyle ou phényle.

Les composés de formule (I) mis en oeuvre préférentiellement répondent aux formules (Ia) et (Ib) dans lesquelles:

- $R_o$ représente un atome d'hydrogène, un radical alkyle ou alkoxy linéaire ou ramifié ayant de 1 à 6 atomes de carbone, de préférence, de 1 à 4 atomes de carbone, un radical cyclohexyle, un radical phényle, un groupe hydroxyle,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

L'invention s'applique plus particulièrement aux composés phénoliques de formule générale (I'):

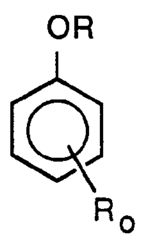

(I')

dans laquelle R et $R_o$, identiques ou différents représentent un atome d'hydrogène, un radical alkyle ayant de 1 à 4 atomes de carbone, un radical cyclohexyle, un radical phényle.

Encore plus préférentiellement, on choisit les composés de formule (I') dans laquelle R représente un atome d'hydrogène et $R_o$ représente un atome d'hydrogène, un radical méthyle, un radical méthoxy.

A titre illustratif de composés phénoliques de formule (I) susceptibles d'être mis en oeuvre dans le procédé de l'invention, on peut mentionner plus particulièrement:

- ceux dans lesquels le reste A répond à la formule (Ia) dans laquelle m et n sont égaux à 0, tels que le phénol ou l'anisole,
- ceux dans lesquels le reste A répond à la formule (Ia) dans laquelle m est égal à O et n est égal à 1, tels que l'o-crésol, le m-crésol, le méthoxy-2 phénol, l'éthyl-2 phénol, l'éthyl-3 phénol, le propyl-2 phénol, le sec-butyl-2 phénol, le tert-butyl-2 phénol, le tert-butyl-3 phénol, le méthoxy-2 phénol, le méthoxy-3 phénol, le salicylate de méthyle, le chloro-2 phénol, le chloro-3 phénol,
- ceux dans lesquels le reste A répond à la formule (Ia) dans laquelle m est égal à O et n est égal à 2, tels que le diméthyl-2,3 phénol, le diméthyl-2,5 phénol, le diméthyl-2,6 phénol, le diméthyl-3,5 phénol, le di-tert butyl-2,6 phénol, le di-tert butyl-3,5 phénol, le dichloro-2,3 phénol, le dichloro-2,5 phénol, le dichloro-2,6 phénol, le dichloro-3,5 phénol,
- ceux dans lesquels le reste A répond à la formule (Ia) dans laquelle m est égal à 0 et n est égal à 3, tels que le triméthyl-2,3,5 phénol, le triméthyl-2,3,6 phénol, le trichloro-2,3,5 phénol, le trichloro-2,3,6 phénol,
- ceux dans lesquels le reste A répond à la formule (Ia) dans laquelle m est égal à 1 et n est égal à 4, tels que l'hydroxy-1 naphtalène,

- ceux dans lesquels le reste A répond à la formule (Ib) dans laquelle p est égal à 1, tels que le phénoxy-2 phénol, le phénoxy-3 phénol.

Le présent procédé convient tout particulièrement bien à la préparation d'hydroquinone et de pyrocatéchol à partir du phénol.

Conformément au procédé de l'invention, on effectue l'hydroxylation du composé phénolique de formule (I), par le peroxyde d'hydrogène, en présence d'un catalyseur qui est un polymère sulfonique et en présence d'un co-catalyseur qui est un composé cétonique de formule (II).

La quantité de polymère sulfonique à mettre en oeuvre est déterminée de telle sorte que le rapport du nombre d'équivalents de protons au nombre de moles de peroxyde d'hydrogène soit compris entre environ $1.10^{-4}$ et environ 1,0.

Une variante préférée du procédé de l'invention consiste à choisir un rapport $H^+/H_2O_2$ compris entre $1.10^{-3}$ et 0,1.

En ce qui concerne le composé cétonique de formule (II), il est mis en oeuvre en quantité catalytique. Généralement, la quantité du composé cétonique de formule (II) exprimée en moles par mole de peroxyde d'hydrogène varie entre $1.10^{-3}$ mole et 10. La quantité de composé cétonique est choisie avantageusement entre 0,05 et 5,0 moles, de préférence entre 0,05 et 1 mole, par mole de peroxyde d'hydrogène.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de $H_2O_2$ et, de préférence, aux environs de 70 %.

La quantité de peroxyde d'hydrogène peut aller jusqu'à 1 mole de $H_2O_2$ pour 1 mole de composé phénolique de formule (I).

Il est cependant préférable pour obtenir un rendement industriellement acceptable d'utiliser un rapport molaire peroxyde d'hydrogène/ composé phénolique de formule (I) de 0,01 à 0,3 et, de préférence, de 0,05 à 0,10.

Afin d'avoir une vitesse de réaction suffisante, on limite la teneur initiale du milieu en eau à 20 % en poids et, de préférence, à 10 % en poids.

Les teneurs pondérales indiquées sont exprimées par rapport au mélange composé phénolique de formule (I)/peroxyde d'hydrogène/eau.

Cette eau initiale correspond à l'eau introduite avec les réactifs et notamment avec le peroxyde d'hydrogène.

Une variante préférée du procédé de l'invention consiste à ajouter un agent complexant des ions métalliques présents dans le milieu car ceux-ci sont préjudiciables au bon déroulement du procédé de l'invention, notamment dans le cas des phénols où les rendements en produits d'hydroxylation sont faibles. Par conséquent, il est préférable d'inhiber l'action des ions métalliques.

Les ions métalliques néfastes au déroulement de l'hydroxylation sont des ions de métaux de transition et plus particulièrement, les ions fer, cuivre, chrome, cobalt, manganèse et vanadium.

Les ions métalliques sont apportés par les réactifs et notamment les composés aromatiques et l'appareillage utilisé. Pour inhiber l'action de ces ions métalliques, il suffit de conduire la réaction en présence d'un ou plusieurs agents complexants stables vis-à-vis du peroxyde d'hydrogène et donnant des complexes ne pouvant être décomposés par les acides forts présents et dans lesquels le métal ne peut plus exercer d'activité chimique.

A titre d'exemples non limitatifs d'agents complexants, on peut faire appel, notamment, aux divers acides phosphoriques tels que, par exemple, l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

On peut également mettre en oeuvre les esters des acides précités et l'on peut mentionner, plus particulièrement, les orthophosphates de mono- ou dialkyle, de mono-ou dicycloalkyle, de mono- ou dialkylaryle, par exemple, le phosphate d'éthyle ou de diéthyle, le phosphate d'hexyle, le phosphate de cyclohexyle, le phosphate de benzyle.

La quantité d'agent complexant dépend de la teneur du milieu réactionnel en ions métalliques.

La quantité d'agent complexant exprimée en nombres de moles d'agent complexant par mole de peroxyde d'hydrogène varie avantageusement entre 0,0001 et 0,01.

Conformément au procédé de l'invention, on réalise l'hydroxylation du composé phénolique de formule (I) à une température qui peut être comprise entre 45°C et 150°C.

Une variante préférée du procédé de l'invention consiste à choisir la température entre 45°C et 75°C.

La réaction est conduite avantageusement sous pression atmosphérique.

Le procédé d'hydroxylation est généralement mis en oeuvre sans solvant autre que celui qui provient des réactifs, comme le solvant du peroxyde d'hydrogène.

Toutefois, il a été trouvé que l'addition d'une certaine quantité d'un solvant organique polaire aprotique présentant certaines caractéristiques de polarité et de basicité permettait d'accroître le rapport para/ortho et plus particuclièrement dans le cas de l'hydroxylation du phénol, le rapport hydroquinone/pyrocatéchine au profit de l'hydroquinone.

Deux classes de solvants organiques conviennent à la mise en oeuvre du procédé de l'invention.

Une première variante du procédé de l'invention consiste donc à effectuer la réaction en présence d'un solvant organique, aprotique, polaire, présentant une polarité telle que sa constante diélectrique est supérieure ou égale à 20 et une basicité telle qu'il possède un "nombre donneur" inférieur à 25.

Plusieurs impératifs président au choix du solvant organique.

Une première caractéristique du solvant organique est qu'il soit aprotique et stable dans le milieu réactionnel.

On entend par solvant aprotique, un solvant qui, dans la théorie de Lewis n'a pas de protons à libérer.

Sont exclus de la présente invention, les solvants qui ne sont pas stables dans le milieu réactionnel et qui se dégradent soit par oxydation, soit par hydrolyse.

Une deuxième caractéristique du solvant organique est qu'il présente une certaine polarité. On choisit, conformément à l'invention, un solvant organique qui présente une constante diélectrique supérieure ou égale à 20. La borne supérieure ne présente aucun caractère critique. On préfère faire appel à un solvant organique ayant une constante diélectrique élevée, de préférence comprise entre 25 et 75.

Afin de déterminer si le solvant organique répond à la condition de constante diélectrique énoncée ci-dessus, on peut se reporter, entre autres, aux tables de l'ouvrage : Techniques of Chemistry, <u>II</u> - Organic solvents - p. 536 et suivantes, 3ème édition (1970).

Une troisième caractéristique du solvant organique est qu'il présente une basicité telle qu'il possède un "nombre donneur" inférieur à 25, de préférence inférieur ou égal à 20. La borne inférieure ne présente aucun caractère critique. On choisit, de préférence, un solvant organique ayant un nombre donneur compris entre 2 et 17.

Pour ce qui des exigences concernant la basicité du solvant organique à mettre en oeuvre, on rappelera que le "nombre donneur" ou "donor number" désigné de manière abrégée DN, donne une indication sur le caractère nucléophile du solvant et révèle son aptitude à donner son doublet.

Dans l'ouvrage de Christian REINHARDT, [Solvents and Solvent Effects in Organic Chemistry - VCH p.19 (1988)], on trouve la définition du "donor number" qui est défini comme le négatif (-ΔH) de l'enthalpie (Kcal/mol) de l'interaction entre le solvant et le pentachlorure d'antimoine, dans une solution diluée de dichloroéthane.

Comme exemples de solvants aprotiques polaires répondant aux caractéristiques précitées, susceptibles d'être mis en oeuvre dans le procédé de l'invention, on peut citer plus particulièrement :

- les composés nitrés tels que le nitrométhane, le nitroéthane, le nitro-1 propane, le nitro-2 propane ou leurs mélanges, le nitrobenzène,
- les nitriles alphatiques ou aromatiques comme l'acétonitrile, le propionitrile, le butanenitrile, l'isobutanenitrile, le benzonitrile, le cyanure de benzyle,
- la tétraméthylène sulfone (sulfolane),
- le carbonate de propylène.

On peut également utiliser un mélange de solvants.

Parmi les solvants précités, on met en oeuvre, préférentiellement, l'acétonitrile.

La quantité de solvant organique tel que précité à mettre en oeuvre est déterminée de telle sorte que le rapport molaire entre le nombre de moles de solvant organique et le nombre de moles de composé phénolique de formule (I) varie entre 0,1 et 2,0, de préférence, entre 0,25 et 1,0.

Une autre variante du procédé de l'invention consiste à effectuer la réaction en présence d'un solvant organique, aprotique, polaire, présentant une polarité telle que sa constante diélectrique est inférieure à environ 20 et une basicité telle qu'il possède un "nombre donneur" supérieur ou égal à 15 et inférieur à 25.

Comme mentionné dans le cas de l'autre classe des solvants précédemment définie, le solvant organique doit être aprotique et stable dans le milieu réactionnel.

Sont également exclus de la présente invention, les solvants qui ne sont pas stables dans le milieu réactionnel et qui se dégradent soit par oxydation, soit par hydrolyse. A titre d'exemples de solvants réactionnels ne convenant pas à l'invention, on peut citer les solvants de type ester dérivés des acides carboxyliques tels que notamment l'acétate de méthyle ou d'éthyle, le phtalate de méthyle ou d'éthyle, le benzoate de méthyle etc...

Une deuxième caractéristique du solvant organique est qu'il présente une certaine polarité. On choisit,

conformément à l'invention, un solvant organique qui présente une constante diélectrique inférieure à environ 20. La borne inférieure ne présente aucun caractère critique. On préfère faire appel à un solvant organique ayant une constante diélectrique faible, de préférence comprise entre 2 et 15.

Une troisième caractéristique du solvant organique est qu'il présente une basicité telle qu'il possède un "nombre donneur" supérieur ou égal à 15 et inférieur à 25. On choisit, de préférence, un solvant organique ayant un nombre donneur compris entre 15 et 25.

Comme exemples de solvants aprotiques polaires répondant aux caractéristiques précitées, susceptibles d'être mis en oeuvre dans le procédé de l'invention, on peut citer plus particulièrement:

- les éther-oxydes aliphatiques, cycloaliphatiques ou aromatiques et, plus particulièrement, l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy- 1,5 oxa-3 pentane), le dioxane, le tétrahydrofuranne,
- les esters neutres phosphoriques tels que, notamment, le phosphate de triméthyle, le phosphate de triéthyle, le phosphate de butyle, le phosphate de triisobutyle, le phosphate de tripentyle,
- le carbonate d'éthylène.

On peut également utiliser un mélange de solvants.

La quantité de solvant organique tel que précité à mettre en oeuvre est déterminée de telle sorte que le rapport molaire entre le nombre de moles de solvant organique et le nombre de moles de composé phénolique de formule (I) varie entre 0,01 et 0,25, de préférence, entre 0,025 et 0,15.

On choisit la quantité de solvant à ajouter en fonction de la basicité du solvant. La quantité de solvant mise en oeuvre sera d'autant plus faible que sa basicité sera plus forte.

En d'autres termes, une quantité située vers la limite inférieure de l'intervalle précédemment défini sera choisie lorsque le solvant présente une basicité élevée.

Conformément à la présente invention, on effectue la réaction d'hydroxylation du composé phénolique de formule (I) par le peroxyde d'hydrogène, en présence d'un polymère sulfonique, d'un composé cétonique de formule (II) et éventuellement en présence d'un solvant organique aprotique polaire tel que précité.

D'un point de vue pratique, le procédé selon l'invention est simple à mettre en oeuvre de façon continue ou discontinue.

D'un manière préférée, on choisit l'ordre des réactifs suivants : on introduit le composé phénolique de formule (I), éventuellement l'agent complexant, le polymère sulfonique puis le composé cétonique de formule (II).

On porte le milieu réactionnel à la température désirée puis, l'on ajoute la solution de peroxyde d'hydrogène, de manière progressive.

En fin de réaction, le catalyseur acide solide est séparé selon les techniques classiques de séparation solide-liquide, de préférence par filtration.

Pour ce qui est du composé phénolique non transformé et du composé cétonique de formule (II), ils sont séparés des produits d'hydroxylation par les moyens usuels, notamment, par distillation et sont renvoyés dans la zone réactionnelle.

Parmi les composés phénoliques de formule (I) qui pourront être mis en oeuvre dans le procédé de l'invention, on peut citer à titre non limitatif, le phénol, l'anisole, l'o-crésol, le m-crésol, le méthoxy-2 phénol.

Le présent procédé convient tout particulièrement bien à la préparation d'hydroquinone et de pyrocatéchine à partir du phénol.

Le procédé, objet de la présente invention, présente de nombreux avantages.

Le procédé est plus simple à mettre en oeuvre puisque le catalyseur est séparé en fin de réaction par une simple filtration et évite donc les opérations de lavage et d'extraction.

Il permet donc lors de la séparation du catalyseur de se dispenser de la mise en oeuvre d'un solvant organique dont l'utilisation conduit toujours à un surcoût, du fait même de son emploi et de la nécessité de le récupérer.

Par ailleurs, le procédé de l'invention procure également un gain énergétique puisqu'il supprime la distillation du solvant d'extraction.

Enfin, notons que le système catalytique hétérogène de l'invention permet non seulement une séparation facile, mais également un recyclage de celui-ci.

En ce qui concerne les résultats atteints par la mise en oeuvre de l'invention, le procédé de l'invention permet d'obtenir des diphénols avec un bon rendement réactionnel et dans les conditions préférentielles, il conduit à une quantité prépondérante d'hydroquinone.

Les exemples qui suivent, illustrent l'invention sans toutefois la limiter.

Dans les exemples, les abréviations suivantes signifient:

$$TT = \frac{\text{nombre de moles de peroxyde d'hydrogène transformées}}{\text{nombre de moles de peroxyde d'hydrogène introduites}} \%$$

$$RT_{HQ} = \frac{\text{nombre de moles d'hydroquinone formées}}{\text{nombre de moles de peroxyde d'hydrogène transformées}} \%$$

$$RT_{PC} = \frac{\text{nombre de moles de pyrocatéchine formées}}{\text{nombre de moles de peroxyde d'hydrogène transformées}} \%$$

EXEMPLES

On donne, ci-après, le protocole opératoire qui va être suivi dans tous les exemples.

Dans un ballon en verre de 100 ml muni d'une agitation centrale, d'un réfrigérant, d'une ampoule de coulée et d'un thermomètre, on charge:

- 47 g (0,50 mol) de phénol,
- x g d'un composé cétonique de formule (II),
- y g d'un polymère sulfonique.

On introduit éventuellement dans certains exemples, z g de solvant aprotique polaire.

Les différentes quantités (x, y et z) peuvent être déterminées à partir des indications données dans les tableaux récapitulatifs.

On porte le mélange réactionnel à la température réactionnelle choisie 75°C, tout en le maintenant sous agitation à 1200 tours/mn.

On introduit par l'intermédiaire de l'ampoule de coulée, en 2 minutes, la solution aqueuse de peroxyde d'hydrogène à 70,5 % en poids en une quantité également précisée dans les tableaux suivants.

On maintient le mélange réactionnel sous agitation, à 75°C, pendant la durée mentionnée dans les tableaux suivants.

On refroidit alors le mélange réactionnel et l'on effectue le dosage des produits de réaction : le peroxyde d'hydrogène résiduel est dosé par iodométrie et les diphénols formés sont dosés par chromatographie liquide, haute performance.

EXEMPLES 1 à 4

Essais comparatifs a et b

Dans cette série d'exemples, on met en oeuvre à titre de catalyseur une résine commercialisée sous la marque TEMEX 50 W et, à titre de co-catalyseur, la benzophénone.

La résine TEMEX 50 W est une résine sulfonique sous forme de Bel ayant une taille de particules de 0,3 à 0,8 mm et ayant une concentration de sites acides de 5,55 milliéquivalents d'$H^+$ par gramme de résine.

Les essais sont conduits selon le protocole opératoire précédemment défini.

Toutes les conditions opératoires et les résultats obtenus sont consignés dans le tableau (I) suivant:

Tableau I

Hydroxylation du phénol par $H_2O_2$/résine TEMEX 50 W/Composé cétonique de formule (II)

| Réf. | Composé cétonique (II) [Rapport molaire composé cétonique (II)/$H_2O_2$] | Solvant organique [rapport molaire solvant organique/phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 1 | benzophénone (0,99) | sans | $5{,}05.10^{-2}$ | $1{,}6.10^{-2}$ | 3H35 | 93 | 34,5 | 35,0 | 0,99 |
| 2 | benzophénone (3,80) | sans | $5{,}2.10^{-2}$ | $1{,}25.10^{-2}$ | 4 H | 95,5 | 39,0 | 37,0 | 1,05 |
| 3 | benzophénone (0,975) | acétonitrile (0,25) | $5{,}15.10^{-2}$ | $2{,}7.10^{-2}$ | 4 H 30 | 91,0 | 36,5 | 33,0 | 1,11 |
| 4 | benzophénone (0,98) | nitrobenzène (0,25) | $5{,}15.10^{-2}$ | $2{,}7.10^{-2}$ | 3 H 30 | 94,0 | 36,5 | 33,5 | 1,09 |
| a | sans | sans | $5{,}25.10^{-2}$ | $1{,}6.10^{-2}$ | 6 H | 99,5 | 14,0 | 32,5 | 0,43 |
| b | acétophénone (1,0) | sans | $5{,}0.10^{-2}$ | $1{,}6.10^{-2}$ | 2 H | 100 | 37,0 | 48,0 | 0,77 |

A titre comparatif, on donne les résultats obtenus lorsque l'on conduit le procédé de l'invention :

- essai a: en l'absence de benzophénone et de solvant organique,
- essai b : en présence d'acétophénone et en l'absence de solvant organique.

L'examen du tableau I met nettement en évidence que la présence de benzophénone permet d'obtenir un bon rendement réactionnel et une quantité plus importante d'hydroquinone que de pyrocatéchine, contrairement au procédé de l'état de la technique.

EXEMPLES 5 à 10

Essais comparatifs c et d

Dans les exemples suivants, on met en oeuvre à titre de catalyseur, une résine commercialisée sous la marque AMBERLYST 15 et, à titre de co-catalyseur, la benzophénone.

La résine AMBERLYST 15 est une résine sulfonique macroporeuse ayant une taille de particules de 0,35 à 1,2 mm, un diamètre moyen des pores de 24 nm et ayant une concentration de sites acides de 4,44 milliéquivalents d'$H^+$ par gramme de résine.

Les essais sont conduits selon le protocole opératoire précédemment défini.

Toutes les conditions opératoires et les résultats obtenus sont consignés dans le tableau (II) suivant :

Tableau II

Hydroxylation du phénol par $H_2O_2$/résine AMBERLYST 15/Composé cétonique de formule (II)

| Réf. | Composé cétonique (II) [Rapport molaire composé cétonique (II)/$H_2O_2$] | Solvant organique [rapport molaire solvant organique/phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 5 | benzophénone (0,99) | sans | $5{,}1.10^{-2}$ | $1{,}65.10^{-2}$ | 3 H | 100 | 30,5 | 35,0 | 0,87 |
| 6 | benzophénone (0,975) | sulfolane (0,25) | $5{,}25.10^{-2}$ | $2{,}8.10^{-2}$ | 4 H | 75,5 | 39,0 | 38,5 | 1,01 |
| 7 | benzophénone (0,985) | nitrobenzène (0,49) | $5{,}0.10^{-2}$ | $5{,}7.10^{-2}$ | 2 H | 98,0 | 26,0 | 30,0 | 0,87 |
| 8 | benzophénone (0,99) | acétonitrile (0,46) | $4{,}7.10^{-2}$ | $2{,}8.10^{-2}$ | 4 H | 99,0 | 29,0 | 30,5 | 0,95 |
| 9 | benzophénone (1,0) | carbonate de propylène (0,25) | $5{,}05.10^{-2}$ | $3{,}0.10^{-2}$ | 1 H 30 | 86,0 | 33,5 | 35,0 | 0,96 |
| 10 | benzophénone (0,98) | carbonate de propylène (0,50) | $5{,}0.10^{-2}$ | $5{,}6.10^{-2}$ | 2 H | 96,0 | 28,0 | 29,0 | 0,97 |
| c | sans | sans | $5{,}3.10^{-2}$ | $1{,}6.10^{-2}$ | 4 H 15 | 100 | 13,0 | 32,0 | 0,41 |
| d | acétophénone (0,94) | sans | $5{,}3.10^{-2}$ | $1{,}6.10^{-2}$ | 1 H 15 | 100 | 33,0 | 45,5 | 0,72 |

EXEMPLE 11

Essais comparafifs e et f

On illustre dans les exemples suivants, la mise en oeuvre, à titre de catalyseur, d'une résine commercialisée sous la marque AMBERLYST 35 et, à titre de co-catalyseur, la benzophénone.

La résine AMBERLYST 35 est une résine polysulfonique macroporeuse ayant une taille de particules de 0,4 à 1,25 mm, un diamètre moyen des pores de 20 nm et ayant une concentration de sites acides de 5,01 milliéquivalents d'$H^+$ par gramme de résine.

Les essais sont conduits selon le protocole opératoire précédemment défini.

Toutes les conditions opératoires et les résultats obtenus sont consignés dans le tableau (III) suivant :

Tableau III

Hydroxylation du phénol par $H_2O_2$/résine AMBERLYST 35/Composé cétonique de formule (II)

| Réf. | Composé cétonique (II) [Rapport molaire composé cétonique (II)/$H_2O_2$] | Solvant organique [rapport molaire solvant organique/phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 11 | benzophénone (0,955) | sans | $4,9.10^{-2}$ | $1,5.10^{-2}$ | 3 H | 100 | 31,5 | 35,5 | 0,89 |
| e | sans | sans | $5,2.10^{-2}$ | $1,6.10^{-2}$ | 5 H | 100 | 15,0 | 33,5 | 0,45 |
| f | acétophénone (1,0) | sans | $5,2.10^{-2}$ | $1,5.10^{-2}$ | 1 H 15 | 100 | 35,0 | 46,5 | 0,75 |

EXEMPLE 12

Essais comparatifs g et h

Dans les exemples suivants, on met en oeuvre à titre de catalyseur, une résine commercialisée sous la marque AMBERLYST 36 et, à titre de co-catalyseur, la benzophénone.

La résine AMBERLYST 36 est une résine polysulfonique macroporeuse ayant une taille de particules de 0,4 à 1,2 mm, un diamètre moyen des pores de 13 nm et ayant une concentration de sites acides de 5,12 milliéquivalents d'$H^+$ par gramme de résine.

Les essais sont conduits selon le protocole opératoire précédemment défini.

Toutes les conditions opératoires et les résultats obtenus sont consignés dans le tableau (IV) suivant :

Tableau IV

Hydroxylation du phénol par $H_2O_2$/résine AMBERLYST 36/Composé cétonique de formule (II)

| Réf. | Composé cétonique (II) [Rapport molaire composé cétonique (II)/$H_2O_2$] | Solvant organique [rapport molaire solvant organique/phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 12 | benzophénone (0,94) | sans | $5{,}2.10^{-2}$ | $1{,}5.10^{-2}$ | 3 H | 100 | 30,5 | 35,0 | 0,87 |
| g | acétophénone (0,975) | sans | $5{,}1.10^{-2}$ | $1{,}6.10^{-2}$ | 1 H 30 | 100 | 28,5 | 38,0 | 0,75 |
| h | sans | sans | $5{,}0.10^{-2}$ | $1{,}6.10^{-2}$ | 5 H 15 | 99,0 | 15,0 | 35,0 | 0,43 |

EXEMPLE 13

Essais comparatifs i et j

On illustre dans les exemples suivants, la mise en oeuvre, à titre de catalyseur, d'une résine commercialisée sous la marque DUOLITE ARC 9359 et à titre de co-catalyseur, la benzophénone.

La résine DUOLITE ARC 9359 est une résine phénol-formol qui porte sur le noyau aromatique, un groupe méthylènesulfonique. Elle a une concentration de sites acides de 2,73 milliéquivalents d'$H^+$ par gramme de résine.

Les essais sont conduits selon le protocole opératoire précédemment défini.

Toutes les conditions opératoires et les résultats obtenus sont consignés dans le tableau (V) suivant :

Tableau V

Hydroxylation du phénol par $H_2O_2$/résine DUOLITE ARC 9359/Composé cétonique de formule (II)

| Réf. | Composé cétonique (II) [Rapport molaire composé cétonique (II)/$H_2O_2$] | Solvant organique [rapport molaire solvant organique/ phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 13 | benzophénone (0,97) | sans | $5,0.10^{-2}$ | $1,55.10^{-2}$ | 2 H | 100 | 29,0 | 35,5 | 0,82 |
| i | acétophénone (1,0) | sans | $5,4.10^{-2}$ | $1,7.10^{-2}$ | 0 H 30 | 99,0 | 35,5 | 48,0 | 0,74 |
| j | sans | sans | $5,4.10^{-2}$ | $1,5.10^{-2}$ | 1 H 30 | 100 | 7,5 | 29,0 | 0,26 |

EXEMPLE 14

Essais comparatifs k ef l

Dans cette série d'exemples, on met en oeuvre à titre de catalyseur, une résine commercialisée sous la marque DOWEX 50 W et, à titre de co-catalyseur, la benzophénone.

La résine DOWEX 50 W est une résine sulfonique sous forme de gel, ayant une taille de particules de 0,04 à 0,07 mm et ayant une concentration de sites acides de 4,51 milliéquivalents d'$H^+$ par gramme de résine.

Les essais sont conduits selon le protocole opératoire précédemment défini.

Toutes les conditions opératoires et les résultats obtenus sont rassemblés dans le tableau (VI) suivant :

Tableau VI

Hydroxylation du phénol par $H_2O_2$/résine DOWEX 50 W/Composé cétonique de formule (II)

| Réf. | Composé cétonique (II) [Rapport molaire composé cétonique (II)/$H_2O_2$] | Solvant organique [rapport molaire* solvant organique/ phénol] | Rapport molaire $H_2O_2$/phénol | Rapport molaire $H^+/H_2O_2$ | Durée | TT | $RT_{HQ}$ | $RT_{PC}$ | Rapport HQ/PC |
|---|---|---|---|---|---|---|---|---|---|
| 14 | benzophénone (0,945) | sans | $5,1.10^{-2}$ | $1,5.10^{-2}$ | 3 H | 100 | 25,0 | 30,0 | 0,83 |
| k | acétophénone (0,935) | sans | $5,6.10^{-2}$ | $1,5.10^{-2}$ | 1 H | 99,5 | 30,0 | 42,5 | 0,71 |
| l | sans | sans | $5,2.10^{-2}$ | $1,6.10^{-2}$ | 3 H | 99,5 | 6,5 | 22,0 | 0,29 |

**Revendications**

**1 -** Procédé d'hydroxylation d'un composé phénolique présentant au moins un atome d'hydrogène en position para du groupe hydroxyle, par réaction dudit composé avec le peroxyde d'hydrogène, en présence d'un catalyseur acide, ledit procédé étant caractérisé par le fait que la réaction est conduite en présence d'une quantité efficace d'un polymère porteur de groupes sulfoniques et d'un composé cétonique répondant à la formule générale (II) :

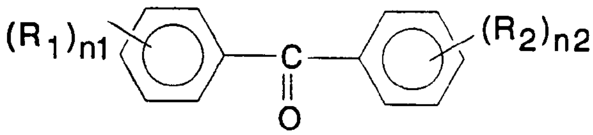

(II)

dans ladite formule (II) :

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position $\alpha$ par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

**2 -** Procédé selon la revendication 1 caractérisé par le fait que la polymère sulfonique est un copolymère styrène-divinylbenzène sulfoné, un copolymère phénol-formaldéhyde sulfoné, un copolymère tétrafluoroéthylène-perfluoro[2-(fluorosulfonyléthoxy)-propyl] vinyl éther.

**3 -** Procédé selon l'une des revendications 1 et 2 caractérisé par le fait que la polymère sulfonique est choisi parmi les résines suivantes : TEMEX 50, AMBERLYST 15, AMBERLYST 35, AMBERLYST 36, DOWEX 50 W; DUOLITE ARC 9359: NAFION.

**4 -** Procédé selon l'une des revendications 1 à 3 caractérisé par le fait que la concentration en sites acides du polymère sulfonique varie entre 1 et 10 milliéquivalents d'ions $H^+$ par gramme de polymère sec, de préférence entre 2 et 7 milliéquivalents d'ions $H^+$ par gramme de polymère sec.

**5 -** Procédé selon l'une des revendications 1 à 4 caractérisé par le fait que le composé cétonique répond à la formule générale (II) dans laquelle $R_1$ et $R_2$, identiques ou différents, représentent :

- des radicaux alkyle linéaires ou ramifiés ayant de 1 à 4 atomes de carbone,
- un radical phényle,
- des radicaux alkoxy $R_3$-O- dans lesquels $R_3$ représente un radical alkyle linéaire ou ramifié ayant de 1 à 4 atomes de carbone ou le radical phényle,
- le groupe hydroxyle,
- un atome de fluor.

**6 -** Procédé selon l'une des revendications 1 à 5 caractérisé par le fait que le composé cétonique répond à la formule générale (II) dans laquelle $R_1$ et $R_2$, identiques ou différents, représentent un atome d'hydrogène ou un groupe électro-donneur en position 4,4' et $n_1$, $n_2$ identiques ou différents sont égaux à 0 ou 1.

**7 -** Procédé selon l'une des revendications 1 à 6 caractérisé par le fait que le composé cétonique répondant à la formule générale (II) dans laquelle $R_1$ et $R_2$, identiques ou différents, représentent un atome d'hydrogène ; un radical méthyle, éthyle, tertiobutyle, phényle ; un radical méthoxy ou éthoxy ; un groupe hydroxyle, de préférence en position 3,3' ou 4,4'.

**8 -** Procédé selon l'une des revendications 1 à 7 caractérisé par le fait que le composé cétonique répond à la formule générale (II) est:

- la benzophénone
- la méthyl-2 benzophénone
- la diméthyl-2,4 benzophénone
- la diméthyl-4,4'benzophénone
- la diméthyl-2,2'benzophénone
- la diméthoxy-4,4'benzophénone
- la fluorénone
- l'hydroxy-4 benzophénone
- la dihydroxy-4-4'benzophénone

- la benzoyl-4 biphényle

**9 -** Procédé selon l'une des revendications 1 à 8 caractérisé par le fait que la quantité de polymère sulfonique à mettre en oeuvre est déterminée de telle sorte que le rapport du nombre d'équivalents de protons au nombre de moles de peroxyde d'hydrogène soit compris entre environ $1.10^{-4}$ et environ 1,0, de préférence entre $1.\ 10^{-3}$ et 0, 1.

**10 -** Procédé selon l'une des revendications 1 à 9 caractérisé par le fait que la quantité du composé cétonique de formule (II) exprimée en moles par mole de peroxyde d'hydrogène varie entre $1.10^{-3}$ mole et 10, de préférence entre 0,05 et 5,0 moles, encore plus préférentiellement entre 0,05 et 1 mole, par mole de peroxyde d'hydrogène.

**11 -** Procédé selon l'une des revendications 1 à 10 caractérisé par le fait que la réaction est conduite en présence d'un solvant organique, aprotique, polaire, présentant une polarité telle que sa constante diélectrique est supérieure ou égale à 20 et une basicité telle qu'il possède un "nombre donneur" inférieur à 25.

**12 -** Procédé selon la revendication 11 caractérisé par le fait que le solvant organique polaire a une constante diélectrique comprise entre 25 et 75.

**13 -** Procédé selon l'une des revendications 11 et 12 caractérisé par le fait que le solvant organique polaire a un nombre donneur inférieur ou égal à 20, de préférence, compris entre 2 et 17.

**14 -** Procédé selon l'une des revendications 11 à 13 caractérisé par le fait que le solvant organique polaire est choisi parmi les composés nitrés, les nitriles aliphatiques ou aromatiques, la tétraméthylène sulfone, le carbonate de propylène.

**15 -** Procédé selon l'une des revendications 11 à 14 caractérisé par le fait que le solvant organique polaire est choisi parmi les solvants suivants : le nitrométhane, le nitroéthane, le nitro-1 propane, le nitro-2 propane ou leurs mélanges, le nitrobenzène, l'acétonitrile, le propionitrile, le butanenitrile, l'isobutanenitrile, le benzonitrile, le cyanure de benzyle, la tétraméthylène sulfone (sulfolane), le carbonate de propylène.

**16 -** Procédé selon l'une des revendications 11 à 15 caractérisé par le fait que la quantité de solvant mis en oeuvre est telle que le rapport molaire entre le nombre de moles de solvant organique et le nombre de moles de composé phénolique de formule (I) varie entre 0,1 et 2,0, de préférence, entre 0,25 et 1,0.

**17 -** Procédé selon l'une des revendications 1 à 10 caractérisé par le fait que la réaction est conduite en présence d'un solvant organique, aprotique, polaire, présentant une polarité telle que sa constante diélectrique est inférieure à environ 20 et une basicité telle qu'il possède un "nombre donneur" supérieur ou égal à 15 et inférieur à 25.

**18 -** Procédé selon la revendication 17 caractérisé par le fait que le solvant organique polaire a une constante diélectrique comprise entre 2 et 15.

**19 -** Procédé selon l'une des revendications 17 et 18 caractérisé par le fait que le solvant organique polaire a un nombre donneur compris entre 15 et 25.

**20 -** Procédé selon l'une des revendications 17 à 19 caractérisé par le fait que le solvant organique polaire est choisi parmi les éther-oxydes aliphatiques, cycloaliphatiques ou aromatiques, les esters neutres phosphoriques, le carbonate d'éthylène.

**21 -** Procédé selon l'une des revendications 17 à 20 caractérisé par le fait que le solvant organique polaire est choisi parmi les solvants suivants : l'oxyde de diéthyle, l'oxyde de dipropyle, l'oxyde de diisopropyle, l'oxyde de dibutyle, le méthyltertiobutyléther, l'oxyde de dipentyle, l'oxyde de diisopentyle, le diméthyléther de l'éthylèneglycol (ou diméthoxy-1,2 éthane), le diméthyléther du diéthylèneglycol (ou diméthoxy-1,5 oxa-3 pentane), le dioxane, le tétrahydrofuranne, le phosphate de triméthyle, le phosphate de triéthyle, le phosphate de butyle, le phosphate de triisobutyle, le phosphate de tripentyle, le carbonate d'éthylène.

**22 -** Procédé selon l'une des revendications 17 à 21 caractérisé par le fait que la quantité de solvant mis en oeuvre est telle que le rapport molaire entre le nombre de moles de solvant organique et le nombre de moles de composé phénolique de formule (I) varie entre 0,01 et 0,25, de préférence, entre 0,025 et 0,15.

**23 -** Procédé selon l'une des revendications 1 à 22 caractérisé par le fait que l'on opère à une température comprise entre 45°C et 150°C, de préférence, entre 45°C et 75°C.

**24 -** Procédé selon l'une des revendications 1 à 23 caractérisé par le fait que le composé phénolique répond à la formule générale (I): dans laquelle

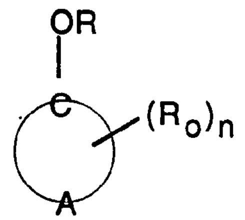

(I)

- A symbolise un reste d'un radical carbocyclique aromatique, monocyclique ou polycyclique ou un radical divalent constitué par un enchaînement de deux ou plusieurs radicaux carbocycliques aromatiques monocycliques,
- R représente un atome d'hydrogène ou un radical hydrocarboné ayant de 1 à 24 atomes de carbone qui peut être un radical aliphatique saturé ou insaturé, linéaire ou ramifié ou un radical cycloaliphatique saturé ou insaturé ou aromatique, monocyclique ou polycyclique,
- $R_o$ représente un ou plusieurs substituants, identiques ou différents,
- n est un nombre inférieur ou égal à 4.

**25 -** Procédé selon la revendication 24 caractérisé par le fait que le composé phénolique répond à la formule (I) dans laquelle:

- le radical R représente l'un des groupes suivants :
  . un atome d'hydrogène
  . un radical alkyle linéaire ou ramifié ayant de 1 à 6 atomes de carbone,
  . un radical cyclohexyle,
  . un radical phényle,
  . un radical benzyle,
- le ou les radicaux $R_o$ représentent l'un des groupes suivants :
  . un atome d'hydrogène
  . un radical alkyle linéaire ou ramifié ayant de 1 à 6 atomes de carbone,
  . un radical alcényle linéaire ou ramifié ayant de 2 à 6 atomes de carbone,
  . un radical alkoxy du type $R_4$-O-, où $R_4$ représente un radical alkyle linéaire ou ramifié ayant de 1 à 6 atomes de carbone,
  . un groupe acyle ayant de 2 à 6 atomes de carbone,
  . un groupe hydroxyle,
  . un groupe $-COOR_5$, où $R_5$ représente un radical alkyle linéaire ou ramifié ayant de 1 à 4 atomes de carbone,
  . un atome d'halogène, de préférence, de fluor, de chlore, de brome,
  . un groupe $-CF_3$.

**26 -** Procédé selon l'une des revendications 1 à 25 caractérisé par le fait que le composé phénolique répond à la formule (I) dans laquelle n est un nombre égal à 0, 1,2 ou 3.

**27 -** Procédé selon l'une des revendications 1 à 26 caractérisé par le fait que le composé phénolique répond à la formule (I) dans laquelle le reste A représente:

- un radical carbocyclique aromatique monocyclique ou polycyclique avec des cycles pouvant former entre eux un système orthocondensé répondant à la formule (Ia) :

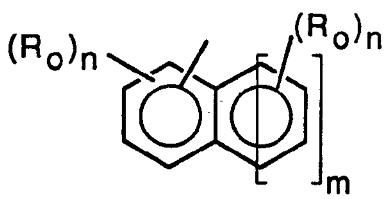

(Ia)

  dans ladite formule (Ia), m représente un nombre égal à 0, 1 ou 2 et les symboles $R_o$ et n identiques ou différents ayant la signification donnée précédemment,
- un radical constitué par un enchaînement de deux ou plusieurs radicaux carbocycliques aromatiques monocycliques répondant à la formule (Ib) :

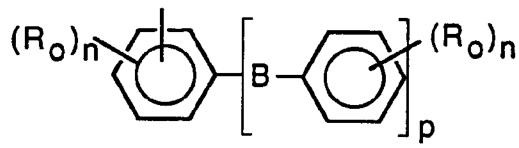

(Ib)

dans ladite formule (Ib), les symboles $R_o$ et n identiques ou différents ont la signification donnée précédemment, p est un nombre égal à 0, 1, 2 ou 3 et B représente :

- un lien valentiel
- un radical alkylène ou alkylidène ayant de 1 à 4 atomes de carbone, de préférence, un radical méthylène ou isopropylidène,
- un des groupes suivants:
  -O- , -COO- , -OCOO-,
  $-SO_2-$,

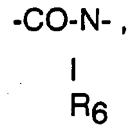

dans ces formules, $R_6$ représente un atome d'hydrogène ou un radical alkyle ayant de 1 à 4 atomes de carbone, un radical cyclohexyle ou phényle.

**28 -** Procédé selon la revendication 27 caractérisé par le fait que le composé phénolique de formule (I) présente un reste A qui répond aux formules (Ia) et (Ib) dans lesquelles:

- $R_o$ représente un atome d'hydrogène, un radical alkyle ou alkoxy linéaire ou ramifié ayant de 1 à 6 atomes de carbone, de préférence, de 1 à 4 atomes de carbone, un radical cyclohexyle, un radical phényle, un groupe hydroxyle,
- B symbolise un lien valentiel, un radical alkylène ou alkylidène ayant de 1 à 4 atomes de carbone ou un atome d'oxygène,
- m est égal à 0 ou 1,
- n est égal à 0, 1 ou 2,
- p est égal à 0 ou 1.

**29 -** Procédé selon la revendication 24 caractérisé parle fait que le composé phénolique répond à la formule générale (I'):

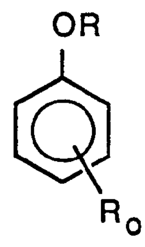

(I')

dans laquelle R et $R_o$, identiques ou différents représentent un atome d'hydrogène, un radical alkyle ayant de 1 à 4 atomes de carbone, un radical cyclohexyle, un radical phényle, et préférentiellement R représente un atome d'hydrogène et $R_o$ représente un atome d'hydrogène, un radical méthyle, un radical méthoxy.

**30 -** Procédé selon l'une des revendications 1 à 29 caractérisé par le fait que le composé phénolique de formule (I) est choisi parmi : le phénol, l'anisole, l'o-crésol, le m-crésol, le méthoxy-2 phénol.

**31 -** Procédé selon l'une des revendications 1 à 30 caractérisé par le fait que le composé phénolique de formule (I) est le phénol.

Office européen des brevets

# RAPPORT DE RECHERCHE EUROPEENNE

Numero de la demande

EP 93 40 0784

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | FR-A-2 266 683 (UBE INDUSTRIES LTD.)<br>* le document en entier; particulièrement page 5, ligne 25-26, page 7, ligne 21-23 et example 27 * | 1-3,6-8, 23-27, 29-31 | C07C37/60<br>C07C39/08 |
| A | FR-A-2 266 684 (UBE INDUSTRIES)<br>* le document en entier; particulièrment page 5, ligne39, page 10, ligne 1-2 et page 16, exemples 26,27 * | 1-3,6-8, 23-27, 29-31 | |
| A | EP-A-0 132 783 (FMC CORPORATION)<br>* le document en entier; particulièrment page 7,ligne 32-34 et page 11, exemple 24 * | 1-31 | |
| A | CHEMICAL ABSTRACTS, vol. 79, no. 5, 6 Août 1973, Columbus, Ohio, US; abstract no. 31665, page 423 ;colonne 2 ;<br>* abrégé *<br>& JP-A-73 036 130 (TOA GOSEI CHEMICAL INDUSTRY CO.)<br>28 Mai 1973 | 1-3, 23-27, 29-31 | DOMAINES TECHNIQUES RECHERCHES (Int. Cl.5)<br>C07C |

Le présent rapport a été établi pour toutes les revendications

| Lieu de la recherche | Date d'achèvement de la recherche | Examinateur |
|---|---|---|
| LA HAYE | 23 JUIN 1993 | HEYWOOD C.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)